Europäisches Patentamt

European Patent Office

Office européen des brevets

(19)

(11) Veröffentlichungsnummer: **0 151 463**
**B1**

(12) **EUROPÄISCHE PATENTSCHRIFT**

(45) Veröffentlichungstag der Patentschrift: **19.09.90**

(21) Anmeldenummer: **85100960.5**

(22) Anmeldetag: **31.01.85**

(51) Int. Cl.⁵: **C 07 K 3/02,** C 07 K 3/28,
G 01 N 33/53, G 01 N 33/68,
C 07 K 15/14, A 61 K 35/38,
A 61 K 35/28, A 61 K 35/28,
A 61 K 37/02

(54) Gewebeprotein PP19, Verfahren zu seiner Gewinnung sowie seine Verwendung.

(30) Priorität: **09.02.84 DE 3404563**

(43) Veröffentlichungstag der Anmeldung:
**14.08.85 Patentblatt 85/33**

(45) Bekanntmachung des Hinweises auf die
Patenterteilung:
**19.09.90 Patentblatt 90/38**

(84) Benannte Vertragsstaaten:
**AT BE CH DE FR GB IT LI LU NL SE**

(56) Entgegenhaltungen:
**EP-A-0 009 715**
**EP-A-0 014 756**
**EP-A-0 037 963**
**DE-A-3 315 000**

(73) Patentinhaber: **BEHRINGWERKE**
**Aktiengesellschaft**
**Postfach 1140**
**D-3550 Marburg 1 (DE)**

(72) Erfinder: **Bohn, Hans, Dr.**
**Oberer Eichenweg 26**
**D-3550 Marburg (DE)**
Erfinder: **Winckler, Wilhelm**
**Untere Bergstrasse 21**
**D-3556 Wenkbach (DE)**

(74) Vertreter: **Klein, Otto, Dr. et al**
**Hoechst AG Zentrale Patentabteilung Postfach**
**80 03 20**
**D-6230 Frankfurt am Main 80 (DE)**

Die Akte enthält technische Angaben, die nach
dem Eingang der Anmeldung eingereicht
wurden und die nicht in dieser Patentschrift
enthalten sind.

Courier Press, Leamington Spa, England.

EP 0 151 463 B1

**Beschreibung**

Die Erfindung betrifft ein Gewebeprotein, das als PP$_{19}$ bezeichnet wird, sowie ein Verfahren zu seiner Gewinnung. PP$_{19}$ kann verwendet werden, um Antiseren herzustellen, die dazu dienen können, PP$_{19}$ im Gewebe und in Körperflüssigkeiten nachzuweisen und zu bestimmen, um Erkrankungen bestimmter Organe zu erkennen, als "Marker" einen Krankheitsverlauf zu überwachen oder eine Therapie zu kontrollieren.

Proteine sind zwar im Stand der Technik bekannt, auch Gewebeproteine, jedoch keines mit den nachfolgend für PP$_{19}$ angegebenen Eigenschaften.

In EP—A—9715 ist das ubiquitäre Gewebsprotein PP8 und ein Verfahren zu seiner Anreicherung beschrieben. Dieses Protein hat einen isoelektrischen Punkt von 4,7 ± 0,3, eine elektrophoretische Beweglichkeit im Bereich der $\alpha_1$-Globuline und weist antigene Eigenschaften auf. Bei Erkrankungen, die mit Gewebszerfall verbunden sind, ist es in erhöhter Konzentration im Blut nachweisbar.

Aus EP—A—37 963 ist das ubiquitäre Gewebsprotein PP9 bekannt, welches außer in der Plazenta in fetalen und adulten Organen nachgewiesen wurde. Dieses Protein hat einen isoelektrischen Punkt im pH-Bereich von 5.0 bis 6.8, eine electrophoretische Beweglichkeit im Bereich der $\beta_1$-Globuline und kann zur Erkennung von Krankheiten, zur Überwachung des Krankheitsverlaufs und zur Kontrolle der Therapie verwendet werden.

Gegenstand der Erfindung ist das Protein PP$_{19}$, gekennzeichnet durch

a) eine elektrophoretische Beweglichkeit im Bereich zwischen der von $\alpha_1$ und $\beta_1$ Globulinen;

b) einen isoelektrischen Punkt zwischen 4,6 und 5,4;

c) einen Sedimentationskoeffizienten $s_{20,w}$ von 3,25 ± 0,25 S;

d) ein in einer Ultrazentrifuge bestimmtes Molekulargewicht von 36 500 ± 4 000; und

e) einen Kohlenhydratanteil von 3,9 ± 1,5 g/100 g (Mannose 0,3 ± 0,2, Fucose 0,2 ± 0,1, Galactose 1,0 ± 0,3, Glukose 0,4 ± 0,2, N-Acetyl-Glukosamin 1,2 ± 0,3, N-Acetyl-Galaktosamin 0,1 ± 0,1, N-Acetyl-Neuraminsäure 0,7 ± 0,3, jeweils g/100 g).

Die Aminosäurenzusammensetzung von PP$_{19}$ ist in der folgenden Tabelle gegeben:

| Aminosäure | Reste pro 100 Reste | Variations-koeffizient |
|---|---|---|
| Lysin | 8,63 | 2,92 |
| Histidin | 1,71 | 14,20 |
| Arginin | 2,73 | 7,71 |
| Asparaginsäure | 11,51 | 3,99 |
| Threonin | 4,20 | 15,75 |
| Serin | 7,48 | 2,26 |
| Glutaminsäure | 11,09 | 3,20 |
| Prolin | 4,12 | 18,29 |
| Glycin | 6,88 | 5,12 |
| Alanin | 7,56 | 3,30 |
| Cystin 1/2 | 2,10 | 22,58 |
| Valin | 7,44 | 3,98 |
| Methionin | 2,39 | 7,24 |
| Isoleucin | 3,40 | 6,38 |
| Leucin | 10,03 | 7,29 |
| Tyrosin | 2,19 | 2,96 |
| Phenylalanin | 5,05 | 2,58 |
| Tryptophan | 1,42 | 15,43 |

**EP 0 151 463 B1**

Zur Erläuterung der kennzeichnenden Merkmale des Gewebsproteins sei folgendes ausgeführt:

Die elektrophoretische Beweglichkeit wurde in der Mikromodifikation mit dem Gerät Microzone R 200 von Beckman Instruments auf Zelluloseazetatfolien (Firma Sartorius) unter Verwendung von Natriumdiethylbarbiturat-Puffer, pH 8,6, bestimmt.

Der isoelektrische Punkt wurde mit einer Säule (440 ml) der Firma LKB, Stockholm, ermittelt. Das Ampholin®-Gemisch hatte einen pH-Bereich von 4,0 bis 6,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner-Technik bei 280 nm bestimmt. Als Lösungsmittel diente Wasser. Die Proteinkonzentration betrug 2 g/l.

Zur Bestimmung des Molekulargewichts in der Ultrazentrifuge wurde die Sedimentationsgleichgewichtsmethode herangezogen. Die Konzentration des Proteins war dazu auf etwa 1,0 O.D. (optische Dichte) eingestellt worden. Als Lösungsmittel diente ein 0,05 mol/l Phosphatpuffer (pH 6,8) der 0,2 mol/l NaCl enthielt. Die Bestimmung wurde bei 9 000 UpM vorgenommen. Die Registrierung erfolgte bei 280 nm unter Einsatz eines photoelektrischen Scanners.

Die Kohlenhydrate wurden wie folgt bestimmt;

Nach Hydrolyse der glykosidischen Bindungen wurden die freigesetzten Neutralzucker als Boratkomplexe über eine Anionaustauschersäule getrennt (Y. C. Lee et al., Anal. Biochem. 27 (1969), 567), im Eluat durch Zumischen von Cu(I)-bicinchoninatreagenz (K. Mopper und M. Gindler, Anal. Biochem. 56 (1973), 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI (1963), 463—465) ermittelt worden.

Die Aminosäurenanalyse wurde nach S. Moore, D. H. Spackman, W. H. Stein, Anal. Chem. 30 (1958), 1185, unter Verwendung eines Flüssigkeitschromatographen Multichrom B der Firma Beckman durchgeführt. Cystin wurde nach Oxydation des Proteins mit Perameisensäure (S. Moore et al., Anal. Chem. 30 (1958), 1185) und nachfolgender Chromatographie (S. Moore, J. Biol. Chem. 238 (1963), 235) als Cysteinsäure bestimmt. Der Tryptophangehalt ist mit der direkten photometrischen Bestimmung nach H. Edelhoch, Biochemistry 6 (1967), 1948, ermittelt worden.

Bei der Untersuchung von Extrakten aus verschiedenen menschlichen Organen wurde $PP_{19}$ in größerer Konzentration in Plazenta und im Magen und in geringerer Konzentration in der Milz mit immunchemischen Methoden nachgewiesen. Extrakte aus anderen Organen des Menschen wie Herz, Lunge, Leber, Niere, Nebenniere, Colon, Rektum, Jejunum und Uterus enthalten dieses Protein nicht oder nur in Spuren. Mit $PP_{19}$ immunchemisch identische oder eng verwandte Proteine konnten auch in Extrakten aus Plazenten von Affen nachgewiesen werden.

Zur Isolierung von $PP_{19}$ können demnach Organe des Menschen oder aber auch von Tieren, in denen dieses Protein vorkommt, verwendet werden. Insbesondere eignen sich dafür ausgewachsene menschliche Plazenten, da sie in reichlicher Menge anfallen und das Protein in genügend großer Konzentration enthalten.

Eine ausgewachsene menschliche Plazenta enthält im Durchschnitt etwa 90 mg $PP_{19}$. Das in der Plazenta enthaltene $PP_{19}$ löst sich bei der Extraktion des Organs mit verdünnten Salz- oder Pufferlösungen, beispielsweise mit physiologischer NaCl-Lösung nur zu etwa 75%. Der Rest des Proteins scheint im Gewebe mit Membranen assoziiert zu sein und geht erst bei Anwendung solubilisierender Agentien, zum Beispiel chaotropen Salzlösungen oder besser noch mit 6 mol/l Harnstofflösung oder 0,5 mol/l Glycin-HCl-Puffer (pH 2,5) oder ionischen Detergenzien, in Lösung.

Zur Gewinnung von $PP_{19}$ kann demnach sowohl der mit verdünnten Salzlösungen erhaltene Proteinextrakt aus Plazenten als auch der, nach Auswaschen der löslichen Bestandteile, aus dem Geweberückstand durch Solubilisierung (zum Beispiel mit 6 mol/l Harnstoff oder saurem Glycin-Puffer) gewonnene Proteinextrakt von Plazenten verwendet werden. Die nach beiden Methoden gewonnenen Proteine sind immunochemisch identisch und stimmen auch in ihren physikalisch-chemischen Eigenschaften weitgehend überein.

$PP_{19}$ hat folgende Eigenschaften, die sich in einem Verfahren zu seiner Isolierung verwenden lassen, indem diesen Eigenschaften entsprechende Maßnahmen getroffen werden:

1) Mit Ammoniumsulfat wird es bei einem pH von 5—8 und 30—70% Sättigung aus wässrigen Lösungen gefällt;

2) Mit wasserlöslichen Acridinbasen, beispielsweise 2-Äthoxy-6,9-diaminoacridinlactat wird es bei einem pH-Wert von 8—9 und einer Konzentration der Base von 4 bis 8 g/l weitgehend präzipitiert. Bei einem pH-Wert von 6,0 und einer Basenkonzentration von 4 g/l fällt es nur zum Teil aus;

3) Bei Zugabe von Äthanol zu einer Lösung in einer physiologischen, oder ähnlich verdünnten, Salzlösung von pH 7 bleibt es bis zu einer Konzentration von 200 ml Alkohol pro 1 Lösung weitgehend im Überstand;

4) Bei einer elektrophoretischen Auftrennung wird es bei einem pH von 7—9 im Bereich zwischen $\alpha_1$ und $\beta_1$ Globulinen gefunden;

5) Bei der isoelektrischen Fokussierung erscheint es im pH-Bereich von 4,6 bis 5,4;

6) Bei einer Gelfiltration mit Sephadex® verhält es sich wie Proteine mit Molekulargewichten von 20.000 bis 60.000;

3

7) Es läßt sich an schwach basische Ionenaustauscher, beispielsweise DEAE-Cellulose oder DEAE-Sephadex, bei einer Leitfähigkeit von etwa 0—2 mS und einem pH-Wert von etwa pH 7 bis 9 binden und kann mit stärker konzentrierten Salzlösungen (10—50 g/l NaCl-Lösungen) eluiert werden;

8) Es kann aus einer wässrigen Lösung durch Immunadsorption angereichert und isoliert werden.

Gegenstand der Erfindung ist demnach auch ein Verfahren zur Gewinnung oder Anreicherung des $PP_{19}$ aus Organen, wie z.B. Plazenta, Magen oder Milz, dadurch gekennzeichnet, daß zunächst durch Behandlung des Organs mit einer Harnstofflösung oder einem Glycin/HCl-Puffer ein Extrakt gewonnen wird, der dann einer oder mehreren der folgenden Maßnahmen unterworfen wird:

a) Fällung des Proteins $PP_{19}$ mit Ammoniumsulfat im pH Bereich von 5 bis 8 und bei 30—70% Sättigung;

b) Fällung des Proteins $PP_{19}$ mit einer wasserlöslichen Acridinbase bei einem pH-Wert von 8 bis 9 und einer Konzentration der Base von 4—8 g/l;

c) Abscheidung eines Teils der Begleitproteine durch Zugabe von Äthanol bei pH 7 bis zu einer Endkonzentration von 200 ml/l Alkohol;

d) präparative Zonenelektrophorese, wobei die Proteinfraktion zwischen den $\alpha_1$ und $\beta_1$ Globulinen gewonnen wird;

e) Gelfiltration oder Ultrafiltration, wobei Proteine im Molekulargewichtsbereich von 20.000 bis 60.000 gewonnen werden;

f) Adsorption an einem schwach basischen Ionenaustauscher und Elution des Proteins $PP_{19}$;

g) immunadsorptive Anreicherung.

Zweckmäßigerweise wird das Verfahren so durchgeführt, daß man Organe, die dieses Protein enthalten, zerkleinert und mit physiologischer Salzlösung wäscht, bis alle löslichen Bestandteile entfernt sind, den Geweberückstand mit beispielsweise einer 6 mol/l Harnstofflösung oder einem 0,5 mol/l Glycin-HCl-Puffer (pH 2,5) extrahiert und den erhaltenen Extrakt nach Neutralisation und gründlicher Dialyse einer oder mehreren der oben beschriebenen Maßnahmen a) bis g) unterwirft.

Neben Ammoniumsulfat können auch andere in der präparativen Biochemie üblicherweise eingesetzte Neutralsalze zur Ausfällung des $PP_{19}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten, seiner Ladung wie seinem Molekulargewicht sind zur Isolierung des Proteins auch andere Maßnahmen brauchbar, die geeignet sind, ein Protein mit den angegebenen Eigenschaften von anderen Proteinen abzutrennen.

Hierzu können die verschiedenen Methoden der präparativen Elektrophorese, der isoelektrischen Fokussierung, der Gelfiltration oder Ultrafiltration oder auch die Eigenschaft des $PP_{19}$, an schwach basische Ionenaustauscher gebunden und hiervon wieder eluiert werden zu können, verwendet werden.

Insbesondere aber ist die spezielle Eigenschaft von $PP_{19}$, teilweise an Membranen gebunden zu sein und davon mit saurem Glycin-Puffer oder mit 6 mol/l Harnstofflösung eluiert werden zu können, zur Isolierung dieses Proteins geeignet.

Insbesondere kann das $PP_{19}$ durch eine zweckmäßige Kombination der genannten Maßnahmen, die eine Anreicherung des $PP_{19}$ oder eine Abtrennung dieses Proteins von anderen Proteinen bewirken, isoliert werden.

Zum Nachweis und zur Bestimmung des $PP_{19}$, etwa in einer Fraktion aus einer Trennoperation, können neben den angegebenen Parametern auch immunchemische Methoden dienen, da $PP_{19}$ antigene Eigenschaften hat.

Ein für diesen Zweck brauchbares Antiserum kann folgendermaßen gewonnen werden:

Bei der Fraktionierung eines Plazentenextraktes mit 2-Äthoxy-6,9-Diaminoacridinlactat und Ammoniumsulfat nach Bohn, H., (Arch. Gynäkol. (1971) *210*, 440) geht $PP_{19}$ zum Teil in die Plazentafraktion II. Trennt man diese Fraktion weiter durch Gelfiltration an Sephadex G—150 auf, so erscheint $PP_{19}$ im Bereich der niedermolekularen Proteine (Molekulargewichte 20.000 bis 60.000). Durch Immunisieren von Kaninchen mit dieser Fraktion erhält man ein polyvalentes Antiserum, das unter anderem Antikörper gegen $PP_{19}$ enthält. Dieses Antiserum kann durch Absorption mit normalem menschlichen Serum, mit Plazentafraktionen, die $PP_{19}$ nicht enthalten, oder mit bestimmten Plazentaproteinen, wie $PP_4$, gegen das Antigen $PP_{19}$ weitgehend spezifisch gemacht werden.

Dieses Antiserum kann einerseits zum immunologischen Nachweis des $PP_{19}$, andererseits zur Herstellung eines Immunadsorbens dienen, das zur Anreicherung und Isolierung von $PP_{19}$ eingesetzt werden kann.

Mit Hilfe des nach Beispiel 1 und insbesondere nach Beispiel 2 der vorliegenden Anmeldung gewonnenen gereinigten $PP_{19}$ lassen sich durch Immunisieren von Tieren nach bekannten Methoden monospezifische Antiseren herstellen.

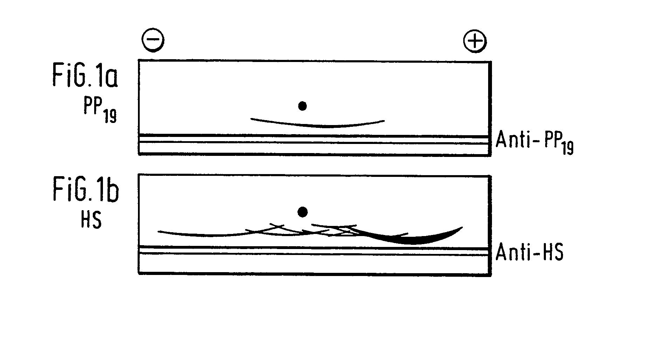

Figur 1a zeigt die immunologische Reaktion von $PP_{19}$ mit einem spezifischen Antiserum vom Kaninchen nach Aauftrennung im elektrischen Feld in Agar-haltigem Gel.

Figur 1b bringt zum Vergleich dazu die Auftrennung der Proteine des Serums, sichtbar gemacht durch deren Immunreaktion mit einem Antiserum von Kaninchen gegen Humanserum (HS).

Zum immunologischen Nachweis von $PP_{19}$ können auch die Geldiffusionstechnik nach Ouchterlony (Schultze and Heremans, Molecular Biology of Human Proteins, Vol. 1, pg. 134) oder, wenn notwendig,

EP 0 151 463 B1

auch empfindlichere Methoden wie Radioimmunoassays oder Enzymimmunoassays herangezogen werden.

Der Nachweis und die Bestimmung von PP$_{19}$ haben diagnostische Bedeutung. PP$_{19}$ ist ein Gewebeprotein, das nur in bestimmten Organen in größerer Konzentration vorkommt. Bei einer Erkrankung dieser Organe kann infolge eines vermehrten Zellzerfalls die Konzentration des Gewebeproteins PP$_{19}$ im Serum oder in anderen Körperflüssigkeiten, beispielsweise im Urin, der Patienten über die Norm ansteigen. Der Nachweis und die Bestimmung von PP$_{19}$ in Körperflüssigkeiten können daher zur Erkennung von Erkrankungen dieser Organe oder auch als Marker zur Überwachung des Krankheitsverlaufs und zur Kontrolle der Therapie eingesetzt werden.

PP$_{19}$ kann also verwendet werden, um Antiseren herzustellen, die dazu dienen können, PP$_{19}$ nachzuweisen und zu bestimmen sowie zum Aufbau von immunchemischen Methoden.

Die Erfindung wird an nachstehenden Beispielen erläutert:

Beispiel 1

A) Extraktion der Plazenten und Fraktionierung des Extraktes mit einer Acridinbase und Ammoniumsulfat

1.000 kg tiefgefrorene menschliche Plazenten wurden im Schneidmischer zerkleinert und mit 1.000 l einer 4 g/l Kochsalzlösung extrahiert. Der Extrakt ist dann, nach Abtrennung des Geweb"rückstandes durch Zentrifugation, mit 200 ml/l Essigsäurelösung auf pH 6,0 eingestellt und unter Rühren mit 200 l einer 30 g/l Lösung von 2-Äthoxy-6,9-Diaminoacridin-Lactat (Hoechst AG) versetzt worden. Der Niederschlag wurde abzentrifugiert und verworfen. Der Überstand wurde mit 10 g/l Betonit A (Fa. Erbslöh and Co., Geisenheim/Rhein) versetzt, durch Zugabe von 2 N NaOH auf pH 7,0 eingestellt und filtriert. Das Filtrat ist unter Rühren langsam mit 300 g/l Ammoniumsulfat versetzt worden; das Plazentaprotein PP$_{19}$ fiel dabei, zusammen mit anderen Proteinen, aus. Der Niederschlag wurde abfiltriert. Es wurden etwa 12 kg einer feuchten Paste erhalten, die im nachfolgenden als Fraktion A bezeichnet wird.

B) Fraktionierung mit Äthanol

500 g der Fraktion A wurden in 400 ml Wasser gelöst und bei 4°C gegen physiologische Kochsalzlösung dialysiert. Nach der Dialyse wurde die Leitfähigkeit der Lösung durch Zugabe von einer 50 g/l NaCl-Lösung auf 15 mS eingestellt. Die Lösung ist dann auf 0°C abgekühlt und unter Rühren langsam mit einem 960 g/l enthaltenden Äthanol bis zu einer Endkonzentration des Alkohols von 200 g/l versetzt worden. Der Niederschlag wurde durch Zentrifugation entfernt. Der Überstand ist zunächst gegen Wasser, dann gegen einen 0,1 mol/l Tris-HCl-Puffer (pH 8,0), der 1 mol/l NaCl und 1 g/l NaN$_3$ enthielt (Pufferlösung II), dialysiert worden. Anschließend wurden die Proteine durch Zugabe von 380 g/l festem Ammoniumsulfat ausgefällt. Der Niederschlag wurde in Wasser gelöst und gegen Pufferlösung II dialysiert. Erhalten wurden etwa 850 ml einer Lösung (Fraktion B), die im Durchschnitt 140 mg PP$_{19}$ enthielt.

C) Anreicherung von PP$_{19}$ durch Immunadsorption

1. Herstellung des Immunadsorbens

340 ml eines Anti-PP$_{19}$-Serums vom Kaninchen wurden gegen 0,02 mol/l Phosphatpuffer (pH 7,0) dialysiert und zur Abtrennung der Immunglobuline an DEAE-Cellulose chromatographiert. Die Immunglobulinfraktion 2,89 g Protein) wurde dann mit 289 g besonders gereinigter Agarose in Kugelform (Sepharose® 4 B der Pharmacia, Uppsala, Schweden), die mit 36,2 g Bromcyan aktiviert worden war, umgesetzt und so kovalent an einen Träger gebunden. Ein geeignetes Verfahren ist beispielsweise beschrieben von Axen el al., Nature *214*, 1302 (1967). Mit Hilfe eines auf diese Weise hergestellten Immunadsorbens konnte das Protein PP$_{19}$ aus seiner Lösung, insbesondere aus PP$_{19}$-angereicherten Plazentenfraktionen, isoliert werden.

2. Durchführung der Immunadsorption

Das Immunadsorbens wurde in Pufferlösung II (0,1 mol/l Tris-HCl-Puffer, pH 8,0, mit 1,0 mol/l NaCl und 1 g/l NaN$_3$) suspendiert, in eine Chromatographiesäule gefüllt (5,0 × 20 cm) und mit Pufferlösung II nachgespült. Dann wurde die halbe Menge der Fraktion B auf die Säule aufgetragen, wobei PP$_{19}$ immunadsorptiv gebunden wurde. Die Säule wurde gründlich mit Puffer II gespült. Anschliessend ist das adsorbierte Protein mit etwa 600 ml 3 mol/l Kaliumrhodanid-Lösung von der Säule eluiert worden. Die PP$_{19}$-haltigen Eluate wurden gegen Pufferlösung II dialysiert und im Ultrafilter auf etwa 10 ml eingeengt. Ausbeute pro Adsorption etwa 6 mg PP$_{19}$.

Das Adsorbens in der Säule wurde unmittelbar nach der Elution von PP$_{19}$ wieder mit Pufferlösung I neutralisiert und gründlich gewaschen. Es ist dann erneut zur immunadsorptiven Bindung von PP$_{19}$ eingesetzt worden.

D) Hochreinigung von PP$_{19}$

Das durch Immunadsorption gewonnene Protein war häufig noch durch unspezifisch gebundene Serumproteine und andere Plazentaproteine verunreinigt. Die Abtrennung der Hauptmenge der Serum-Begleitproteine gelang durch Gelfiltration an Sephadex G—100. Die restlichen Begleitproteine wurden dann durch inverse oder negative Immunadsorption, das heißt mit Hilfe von trägergebundenen Antikörpern gegen die noch als Verunreinigung vorliegenden Proteine, entfernt. Im wesentlichen handelte es

5

sich dabei um Immunglobuline und die Plazentagewebeproteine PP$_7$, PP$_9$, PP$_{11}$ und PP$_{13}$ sowie um ein Erythrozytenprotein, genannt EP$_3$.

Die Gewinnung der Plazenta-Gewebeproteine PP$_7$, PP$_9$, PP$_{11}$ und PP$_{13}$ wurde bereits früher beschrieben (DE—OS 26 40 387, EP—A 0 037 963, EP—A 0 029 191, DE—OS 32 30 996). Antiseren gegen diese Proteine sind durch Immunisierung von Kaninchen mit diesen Proteinen gewonnen worden.

Das als Veranreinigung in der PP$_{19}$ Fraktion aufgefundene Erythrozytenprotein EP$_3$ hat ein wesentlich kleineres Molekulargewicht (etwa 15.000) als PP$_{19}$ und wird daher bereits bei der Gelfiltration an Sephadex G—100 größtenteils vom PP$_{19}$ abgetrennt. Es erscheint in den niedermolekularen Fraktionen (MG unter 20.000) fast völlig frei von anderen Proteinen. Durch Immunisieren von Kaninchen mit dieser Nebenfraktion wurden Antiseren gegen das Erythrozytenprotein gewonnen und zur Herstellung eines entsprechenden Immunadsorbens verwendet.

## Beispiel 2

### A) Zerkleinerung und Waschung der Plazenten

Zur Isolierung des membran-assoziierten Teils von PP$_{19}$ wurden reife menschliche Plazenten, wie sie bei einer Entbindung anfallen, im gefrorenen Zustand mit einem Schneidmischer zerkleinert und in dieser Form bei −20°C bis zur Verwendung aufbewahrt. Durch Waschung mit physiologischer NaCl-Lösung wurden zunächst alle löslichen Gewebsproteine entfernt. Dazu wurden 500 g des zerkleinerten Plazentengewebes mit 700 ml NaCl-Lösung versetzt, kurz homogenisiert, dann mehrere Stunden bei 4°C gerührt und schließlich zentrifugiert. Der Überstand wurde verworfen, der Rückstand erneut mit 700 ml NaCl-Lösung mehrere Stunden gerührt und wieder zentrifugiert. Dieser Waschvorgang wurde insgesamt 6 mal wiederholt. Auf diese Weise wurde das Plazentagewebe von löslichen Bestandteilen weitgehend befreit.

### B) Extraktion des Plazentagewebes mit Triton® X—100

Der Geweberückstand wurde dreimal mit je 700 ml einer Lösung von 2 ml Polyethylenglycol-p-isooctylphenylether (Triton® X—100) in 100 ml Wasser extrahiert, wobei jeweils 20 Stunden bei 4°C gerührt und dann abzentrifugiert wurde. Die Behandlung mit dem nichtionischen Detergens Triton® X—100 diente zur Entfernung der membran-assoziierten Proteine MP$_1$ und MP$_2$ (DE—OS 33 14 293 und 33 34 405). Der membran-assoziierte Anteil des Proteins PP$_{19}$ geht dabei kaum in Lösung.

### C) Solubilisierung von PP$_{19}$ mit saurem Glycinpuffer

Zur Solubilisierung von PP$_{19}$ wurde der Geweberückstand nach der Behandlung mit Triton® X—100 zweimal mit je 500 ml eines 0,5 mol/l Glycin-HCl-Puffers (pH 2,5) 2 Stunden bei 4°C gerührt und abzentrifugiert. Die Extrakte sind dann gegen einen 0,1 mol/l Tris-HCl-Puffer (pH 8,0), der 1 mol/l NaCl und 1 g/l Natriumazid enthielt (Pufferlösung II), dialysiert worden. Nach der Dialyse wurden die Lösungen auf einem Ultrafilter (Fa. Amicon) unter Verwendung von PM—10 Membranen auf jeweils etwa 100 ml eingeengt. Die Extrakte aus dem Rückstand von ursprünglich 500 g Plazentagewebe enthielten im Durchschnitt insgesamt etwa 20 mg PP$_{19}$ (Fraktion 2A).

### D) Anreicherung von PP$_{19}$ durch Immunadsorption

Die Herstellung des Immunadsorbens und die Durchführung der Immunadsorption erfolgten wie in Beispiel 1 beschrieben. Zur Adsorption wurde aber jetzt die durch Solubilisierung mit Glycinpuffer aus dem Plazentagewebe erhaltene PP$_{19}$-Lösung (Fraktion 2A) eingesetzt. Die Elution des adsorbierten Proteins von der Säule erfolgte mit 6 mol/l Harnstofflösung.

### E) Hochreinigung von PP$_{19}$

Zur Hochreinigung des durch Immunadsorption gewonnenen Proteins genügte im allgemeinen eine Gelfiltration an Acrylamidagarose AcA 34. Eventuell noch in Spuren vorhandene andere Plazentagewebeproteine wurden durch inverse Immunadsorption entfernt.

## Patentansprüche

1. Protein PP$_{19}$, gekennzeichnet durch
a) eine elektrophoretische Beweglichkeit im Bereich zwischen der von $\alpha_1$ und $\beta_1$ Globulinen;
b) einen isoelektrischen Punkt zwischen 4,6 und 5,4;
c) einen Sedimentationskoeffizienten $s_{20,w}$ von 3,25 ± 0,25 S;
d) ein in einer Ultrazentrifuge bestimmtes Molekulargewicht von 36 500 ± 4 000; und
e) einen Kohlenhydratanteil von 3,9 ± 1,5 g/100 g (Mannose 0,3 ± 0,2, Fucose 0,2 ± 0,1, Galactose 1,0 ± 0,3, Glukose 0,4 ± 0,2, N-Acetyl-Glukosamin 1,2 ± 0,3, N-Acetyl-Galaktosamin 0,1 ± 0,1, N-Acetyl-Neuraminsäure 0,7 ± 0,3, jeweils g/100 g);
f) eine Aminosäurenzusammensetzung gemäß folgender Tabelle:

| Aminosäure | Reste pro 100 Reste | Variations- koeffizient |
|---|---|---|
| Lysin | 8,63 | 2,92 |
| Histidin | 1,71 | 14,20 |
| Arginin | 2,73 | 7,71 |
| Asparaginsäure | 11,51 | 3,99 |
| Threonin | 4,20 | 15,75 |
| Serin | 7,48 | 2,26 |
| Glutaminsäure | 11,09 | 3,20 |
| Prolin | 4,12 | 18,29 |
| Glycin | 6,88 | 5,12 |
| Alanin | 7,56 | 3,30 |
| Cystin 1/2 | 2,10 | 22,58 |
| Valin | 7,44 | 3,98 |
| Methionin | 2,39 | 7,24 |
| Isoleucin | 3,40 | 6,38 |
| Leucin | 10,03 | 7,29 |
| Tyrosin | 2,19 | 2,96 |
| Phenylalanin | 5,05 | 2,58 |
| Tryptophan | 1,42 | 15,43 |

2. Verfahren zur Gewinnung oder Anreicherung des $PP_{19}$, nach Anspruch 1, aus Organen, wie z.B. Plazenta, Magen oder Milz, dadurch gekennzeichnet, daß zunächst durch Behandlung des Organs mit einer Harnstofflösung oder einem Glycin/HCl-Puffer ein Extrakt gewonnen wird, der dann einer oder mehreren der folgenden Maßnahmen unterworfen wird:

a) Fällung des Proteins $PP_{19}$ mit Ammoniumsulfat im pH- Bereich von 5 bis 8 und bei 30—70% Sättigung;

b) Fällung des Proteins $PP_{19}$ mit einer wasserlöslichen Acridinbase bei einem pH-Wert von 8 bis 9 und einer Konzentration der Base von 4—8 g/l;

c) Abscheidung eines Teils der Begleitproteine durch Zugabe von Äthanol bei pH 7 bis zu einer Endkonzentration von 200 ml/l Alkohol;

d) präparative Zonenelektrophorese, wobei die Proteinfraktion zwischen den $α_1$ und $β_1$ Globulinen gewonnen wird;

e) Gelfiltration oder Ultrafiltration, wobei Proteine im Molekulargewichtsbereich von 20.000 bis 60.000 gewonnen werden;

f) Adsorption an einem schwach basischen Ionenaustauscher und Elution des Protein $PP_{19}$;

g) immunadsorptive Anreicherung.

3. Verwendung des Proteins $PP_{19}$ nach Anspruch 1 zur Gewinnung eines Antiserums und zum Aufbau von immunchemischen Methoden zum Nachweis und zur Bestimmung dieses Proteins, zur Erkennung von Erkrankungen, zur Verlaufskontrolle von Erkrankungen oder zur Kontrolle einer Therapie.

**Revendications**

1. Protéine $PP_{19}$, caractérisée par

a) une mobilité électrophorétique dans l'intervalle compris entre celle des $α_1$-globulines et celle des $β_1$- globulines;

EP 0 151 463 B1

b) un point isoélectrique entre 4,6 et 5,4;

c) un coefficient de sédimentation $S_{20,w}$ de 3,25 ± 0,25 S;

d) une masse moléculaire, déterminée en ultracentrifugeuse, de 36500 ± 4000; et

e) un pourcentage d'hydrates de carbone de 3,9 ± 1,5 g/100 g (mannose 0,3 ± 0,2, fucose 0,2 ± 0,1, galactose 1,0 ± 0,3, glucose 0,4 ± 0,2, N-acétylglucosamine 1,2 ± 0,3, N-acétylgalactosamine 0,1 ± 0,1, acide N-acétylneuraminique 0,7 ± 0,3, chaque fois en g/100 g).

f) une composition en acides aminés selon le tableau suivant:

| Acide aminé | Restes pour 100 restes | Coefficient de variation |
|---|---|---|
| Lysine | 8,63 | 2,92 |
| Histidine | 1,71 | 14,20 |
| Arginine | 2,73 | 7,71 |
| Acide aspartique | 11,51 | 3,99 |
| Thréonine | 4,20 | 15,75 |
| Sérine | 7,48 | 2,26 |
| Acide glutamique | 11,09 | 3,20 |
| Proline | 4,12 | 18,29 |
| Glycine | 6,88 | 5,12 |
| Alanine | 7,56 | 3,30 |
| Cystine 1/2 | 2,10 | 22,58 |
| Valine | 7,44 | 3,98 |
| Méthionine | 2,39 | 7,24 |
| Isoleucine | 3,40 | 6,38 |
| Leucine | 10,03 | 7,29 |
| Tyrosine | 2,19 | 2,96 |
| Phénylalanine | 5,05 | 2,58 |
| Tryptophane | 1,42 | 15,43 |

2. Procédé d'obtention ou d'enrichissement de $PP_{19}$, selon la revendication 1, à partir d'organes, tels que p.ex. placenta, estomac ou rate, caractérisé en ce que l'on obtient d'abord, par traitement de l'organe avec une solution d'urée ou un tampon glycine/HCl, un extrait qui peut être soumis à une ou plusieurs des dispositions suivantes:

a) précipitation de la protéine $PP_{19}$ avec du sulfate d'ammonium dans un intervalle de pH de 5 à 8 et à une concentration de 30 à 70%;

b) précipitation de la protéine $PP_{19}$ avec une base d'acridine hydrosoluble à un pH de 8 à 9 et une concentration de la base de 4 à 8 g/l;

c) séparation d'une partie des protéines d'accompagnement par addition d'éthanol à pH 7 jusqu'à une concentration finale de 200 ml/l d'alcool;

d) électrophorèse préparative de zone, pour obtenir la fraction protéinique entre les globulines $\alpha_1$ et $\beta_1$;

e) filtration sur gel ou ultrafiltration, pour obtenir les protéines dans l'intervalle de masses moléculaires 20000 à 60000;

f) adsorption sur échangeur d'ions faiblement basique et élution de la protéine $PP_{19}$;

g) enrichissement par immunoadsorption.

3. Utilisation de la protéine $PP_{19}$ selon la revendication 1, pour l'obtention d'un antisérum et pour l'élaboration de techniques immunochimiques pour l'identification et le dosage de cette protéine, pour le diagnostic de maladies, pour le contrôle du déroulement de maladies ou pour le contrôle d'une thérapeutique.

8

## EP 0 151 463 B1

**Claims**

1. Protein PP$_{19}$ having the following characteristics:
   a) an electrophoretic mobility in the region between that of $\alpha_1$ and of $\beta_1$ globulins;
   b) an isoelectric point between 4.6 and 5.4;
   c) a sedimentation coefficient $S_{20,w}$ of $3.25 \pm 0.25$ S;
   d) a molecular weight determined in an ultracentrifuge of $36,500 \pm 4,000$; and
   e) a carbohydrate content of $3.9 \pm 1.5$ g/100 g (mannose $0.3 \pm 0.2$, fucose $0.2 \pm 0.1$, galactose $1.0 \pm 0.3$, glucose $0.4 \pm 0.2$, N-acetylglucosamine $1.2 \pm 0.3$, N-acetylgalactosamine $0.1 \pm 0.1$, and N-acetyl-neuraminic acid $0.7 \pm 0.3$, each g/100 g);
   f) an amino acid composition as defined in the table below:

| Amino acid | Residues per 100 residues | Coefficient of variation |
|---|---|---|
| lysine | 8.63 | 2.92 |
| histidine | 1.71 | 14.20 |
| arginine | 2.73 | 7.71 |
| aspartic acid | 11.51 | 3.99 |
| threonine | 4.20 | 15.75 |
| serine | 7.48 | 2.26 |
| glutamic acid | 11.09 | 3.20 |
| proline | 4.12 | 18.29 |
| glycine | 6.88 | 5.12 |
| alanine | 7.56 | 3.30 |
| cystine 1/2 | 2.10 | 22.58 |
| valine | 7.44 | 3.98 |
| methionine | 2.39 | 7.24 |
| isoleucine | 3.40 | 6.38 |
| leucine | 10.03 | 7.29 |
| tyrosine | 2.19 | 2.96 |
| phenylalanine | 5.05 | 2.58 |
| tryptophan | 1.42 | 15.43 |

2. A process for obtaining or enriching PP$_{19}$, as claimed in claim 1, such as, for example, from organs, placenta, stomach or spleen, which comprises initially treating the organ with a urea solution or a glycine/HCl buffer to obtain an extract which is then subjected to one or more of the following measures:
   a) Precipitation of the protein PP$_{19}$ with ammonium sulfate in the pH range 5 to 8 and at 30—70% saturation;
   b) Precipitation of the protein PP$_{19}$ using a water-soluble acridine base at a pH of 8 to 9 and a concentration of the base of 4—8 g/l;
   c) Separation out of part of the accompanying proteins by addition of ethanol at pH 7 up to a final concentration of 200 ml/l alcohol;
   d) Preparative zone electrophoresis, where the protein fraction between $\alpha_1$ and $\beta_1$ globulins is obtained;
   e) Gel filtration or ultrafiltration, where proteins in the molecular weight range 20,000 to 60,000 are obtained;

9

f) Absorption onto a weakly basic ion exchanger and elution of the protein $PP_{19}$;

g) Enrichment by immunoadsorption.

3. The use of the protein $PP_{19}$ as claimed in claim 1 for obtaining an antiserum and for designing immunochemical methods for the detection and determination of this protein, for the diagnosis of diseases, for monitoring the course of diseases or for monitoring a treatment.

FiG.1a
PP₁₉

FiG.1b
HS